(19) Europäisches Patentamt
European Patent Office
Office européen des brevets

(11) **EP 3 675 036 A1**

(12) **EUROPEAN PATENT APPLICATION**

(43) Date of publication:
**01.07.2020 Bulletin 2020/27**

(51) Int Cl.:
**G06T 7/30** $^{(2017.01)}$

(21) Application number: **18306869.1**

(22) Date of filing: **28.12.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Trophy**
**77435 Marne La Vallée Cedex 2 (FR)**

(72) Inventors:
• **JACOB, Jean-Pascal**
**77435 MARNE LA VALLEE CEDEX 2 (FR)**

• **REYNARD, Delphine**
**77435 MARNE LA VALLEE CEDEX 2 (FR)**
• **RIPOCHE, Xavier**
**77435 MARNE LA VALLEE CEDEX 2 (FR)**
• **ROBERTS, Gaël**
**77435 MARNE LA VALLEE CEDEX 2 (FR)**
• **CAPRON-RICHARD, Sabrina**
**77435 MARNE LA VALLEE CEDEX 2 (FR)**

(74) Representative: **Santarelli**
**49, avenue des Champs-Elysées**
**75008 Paris (FR)**

(54) **3D SEGMENTATION FOR MANDIBLE AND MAXILLA**

(57)     A method for registering a 3D mesh and a 3D volume image of a patient's teeth obtains a 3D volume image of the patient's teeth in an occlusion position, the 3D volume image containing at least one jaw portion. A contour surface is generated to form the 3D mesh corresponding to at least one of lingual and vestibular surfaces of the teeth from the 3D volume image. A registered mesh is provided in an occlusion position, the registered mesh having at least one of the vestibular and lingual surfaces. The registered mesh is registered with the generated contour surface content and displayed with the 3D volume image.

FIG. 10A

Description

TECHNICAL FIELD

The disclosure relates generally to dental imaging and more particularly relates to methods and apparatus for segmentation of intraoral features.

BACKGROUND

A 3-dimensional (3D) or volume x-ray image can be of significant value for diagnosis and treatment of teeth and supporting structures. A volume x-ray image for this purpose is formed by combining image data from two or more individual 2D projection images, obtained within a short time of each other and with a well-defined angular and positional geometry between each projection image and the subject tooth and between each projection image and the other projection images. Cone-Beam Computed Tomography (CBCT) is one established method for obtaining a volume image of dental structures from multiple projection images. In CBCT imaging, an image detector and a radiation source orbit a subject and obtain a series of x-ray projection images at small angular increments. The information obtained is then used to synthesize a volume image that faithfully represents the imaged subject to within the available resolution of the system, so that the volume image that is formed can then be viewed from any number of angles. Commercially available CBCT apparatus for dental applications include the CS 8100 3D System from Carestream Health, Inc., Rochester, NY.

For extraoral CBCT imaging, it is often useful to segment the maxilla and mandible so that upper and lower jaw features can be viewed and manipulated separately. The capability for accurate segmentation of maxilla and mandible has particular advantages for assessing how these structures work together.

One complication with significant impact on jaw structures segmentation relates to image acquisition practices that acquire radiographic projection images with the patient's teeth in occlusion. Having upper and lower teeth in occlusion is most favorable for maintaining the mouth in a stable position during the x-ray imaging procedure. However, with the mouth closed and with some partial overlap of maxillary and mandibular teeth, it can be challenging to algorithmically identify the full tooth structure with a sufficiently high degree of certainty.

Thus, it can be appreciated that there would be value in a mandible/maxilla segmentation approach for 3D volume data that operates with teeth in occlusion for volume imaging but allows the patient's mouth to be open during surface contour scanning.

SUMMARY

An object of the present invention is to advance the art of intraoral radiography by providing apparatus and methods for generating a volume image from a small number of x-ray images obtained by an intraoral imaging detector.

These objects are given only by way of illustrative example, and such objects may be exemplary of one or more embodiments of the invention. Other desirable objectives and advantages inherently achieved by the disclosed methods may occur or become apparent to those skilled in the art. The invention is defined by the appended claims.

According to one aspect of the disclosure, there is provided a method for registering a 3D mesh and a 3D volume image of a patient's teeth comprising:

a) obtaining a 3D volume image of the patient's teeth in an occlusion position, the 3D volume image containing at least one jaw portion;
b) generating contour surface content corresponding to at least one of lingual and vestibular surfaces of the teeth from the 3D volume image;
c) providing a registered 3D mesh in an occlusion position, the registered mesh comprising at least a portion of a maxillary jaw and a mandibular jaw, each of the jaw comprising at least one of the vestibular and lingual surfaces;
d) registering the registered 3D mesh with the generated contour surface content.

BRIEF DESCRIPTION OF THE DRAWINGS

The foregoing and other objects, features, and advantages of the invention will be apparent from the following more particular description of the embodiments of the invention, as illustrated in the accompanying drawings.

The elements of the drawings are not necessarily to scale relative to each other.

FIG. 1 is a schematic diagram showing an image apparatus for CBCT imaging of the head.
FIG. 2 is a schematic diagram that shows how CBCT imaging acquires multiple radiographic projection images for reconstruction of the 3D volume image.
FIG. 3 is a schematic diagram that shows a surface contour imaging apparatus for obtaining a 3D view from a succession of reflectance images according to an embodiment of the present disclosure.
FIG. 4 is a schematic diagram showing an imaging apparatus that can operate as a video camera for polychromatic reflectance image data capture as well as a scanner for executing related projecting and imaging functions.
FIG. 5 is a schematic diagram that shows how patterned light is used for obtaining surface contour information by a scanner using a handheld camera or other portable imaging device.
FIG. 6 shows surface imaging using a structured light pattern with multiple lines of light.

FIG. 7 shows a 3D mesh generated from a succession of structured light images.

FIG. 8A shows an exemplary 3D volume image.

FIG. 8B shows a 3D mesh or point cloud image.

FIG. 9A is a logic flow diagram that shows the overall sequence for registering a 3D mesh or point cloud for a patient with a corresponding 3D volume image reconstruction from CBCT.

FIG. 9B is a logic flow diagram that shows a detailed sequence for registering a 3D mesh or point cloud for a patient with a corresponding 3D volume image reconstruction from CBCT.

FIG. 10A shows features of a sub-mesh extraction sequence described in the detailed sequence shown in FIG. 9B.

FIG. 10B shows a single parabola from a slice, shown in outline form.

FIG. 11A is a perspective view that shows multiple points of interest along a surface contour point cloud or 3D mesh image of the mandible.

FIG. 11B shows a parabola calculated using the points of FIG. 11A.

FIG. 11C shows an example with vestibular surface portions of the maxillary mesh or point cloud identified.

FIG. 11D shows extracted vestibular mandible and maxillar 3D meshes.

FIGs. 11E and 11F show parabola generation for a single slice of the 3D mesh image.

FIG. 11G shows coarse alignment of features using parabola stacking according to an embodiment.

FIG. 12A shows coarse alignment of a mesh sub-part to the VOI.

FIG. 12B shows fine alignment using slight translation and rotation.

FIGs. 13A and 13B show views of a completed registration of mesh combined vestibular surfaces to volume vestibular surfaces according to an embodiment of the present disclosure.

## DETAILED DESCRIPTION OF THE EMBODIMENTS

The following is a detailed description of the preferred embodiments, reference being made to the drawings in which the same reference numerals identify the same elements of structure in each of the several figures.

Where they are used in the context of the present disclosure, the terms "first", "second", and so on, do not necessarily denote any ordinal, sequential, or priority relation, but are simply used to more clearly distinguish one step, element, or set of elements from another, unless specified otherwise.

As used herein, the term "energizable" relates to a device or set of components that perform an indicated function upon receiving power and, optionally, upon receiving an enabling signal.

In the context of the present disclosure, the terms "viewer", "operator", and "user" are considered to be equivalent and refer to the viewing practitioner, technician, or other person who views and manipulates an image, such as a dental image, on a display monitor. An "operator instruction" or "viewer instruction" is obtained from explicit commands entered by the viewer, such as by clicking a button on a camera or by using a computer mouse or by touch screen or keyboard entry.

In the context of the present disclosure, the phrase "in signal communication" indicates that two or more devices and/or components are capable of communicating with each other via signals that travel over some type of signal path. Signal communication may be wired or wireless. The signals may be communication, power, data, or energy signals. The signal paths may include physical, electrical, magnetic, electromagnetic, optical, wired, and/or wireless connections between the first device and/or component and second device and/or component. The signal paths may also include additional devices and/or components between the first device and/or component and second device and/or component.

In the present disclosure, the term "detector" refers to the element that is placed in the patient's mouth, receives radiation, and provides the image content. Such a detector is a digital detector that provides the x-ray image data directly to an imaging system.

In the context of the present disclosure, the terms "pixel" and "voxel" may be used interchangeably to describe an individual digital image data element, that is, a single value representing a measured image signal intensity. Conventionally an individual digital image data element is referred to as a voxel for 3-dimensional volume images and a pixel for 2-dimensional images. Volume images, such as those from CT or CBCT apparatus, are formed by obtaining multiple 2D images of pixels, taken at different relative angles, then combining the image data to form corresponding 3D voxels. For the purposes of the description herein, the terms voxel and pixel can generally be considered equivalent, describing an image elemental datum that is capable of having a range of numerical values. Voxels and pixels have the attributes of both spatial location and image data code value.

For general description and background on CT imaging, reference is hereby made to US 8670521 entitled "Method for Generating an Intraoral Volume Image" by Bothorel et al., commonly assigned.

The terms "3D model", "point cloud", and "mesh" may be used synonymously in the context of the present disclosure for image structures that visually represent the 3D surface contour of imaged teeth. A dense point cloud is formed using techniques familiar to those skilled in the volume imaging arts for surface contour representation and relates generally to methods that identify points in space corresponding to surface features. A dense point cloud can be generated, for example, using the reconstructed contour data from one or more reflectance images. A mesh can be generated using the same acquired surface contour to identify vertices that serve as the basis for a polygon model for tooth and gum surfaces. The mesh and point cloud representations for a 3D surface can have the same visual appearance depending on magnification; computed coordinates for vertices of the mesh and particular points in the point cloud, however, need not be identical.

Overview of dental CBCT apparatus

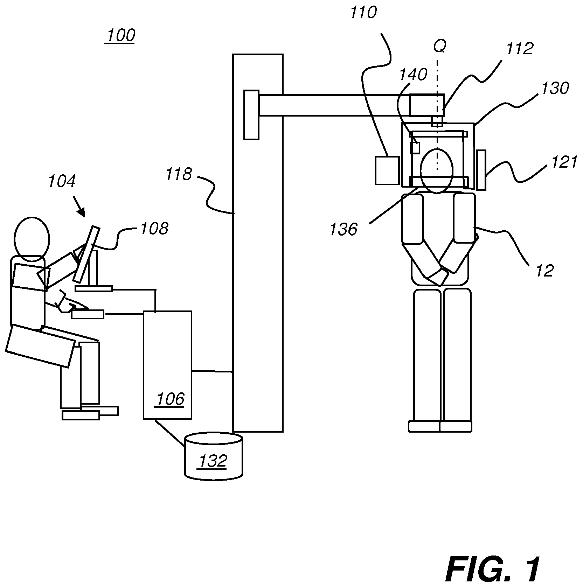

The schematic diagram of FIG. 1 shows an imaging apparatus 100 for 3D CBCT cephalometric imaging, including dental imaging. For imaging a patient 12, a succession of multiple 2D projection images is obtained and processed using imaging apparatus 100. A rotatable mount 130 is provided on a column 118, preferably adjustable in height to suit the size of patient 12. Mount 130 maintains an x-ray source 110 and a radiation sensor 121 on opposite sides of the head of patient 12 and rotates to orbit source 110 and sensor 121 in a scan pattern about the head. Mount 130 rotates about an axis Q that corresponds to a central portion of the patient's head, so that components attached to mount 130 orbit the head. Sensor 121, a digital sensor, is coupled to mount 130, opposite x-ray source 110 that emits a radiation pattern suitable for CBCT volume imaging. An optional head support 136, such as a chin rest or bite element, provides stabilization of the patient's head during image acquisition. A computer 106 has an operator interface 104 and a display 108 for accepting operator commands and for display of volume images of the orthodontia image data obtained by imaging apparatus 100. Computer 106 is in signal communication with sensor 121 for obtaining image data and provides signals for control of source 110 and, optionally, for control of a rotational actuator 112 for mount 130 components. Computer 106 is also in signal communication with a memory 132 for storing image data. An optional alignment apparatus 140 is provided to assist in proper alignment of the patient's head for the imaging process.

Volume image reconstruction from multiple projection images

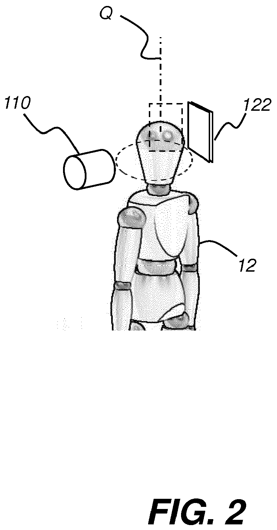

The schematic diagram of FIG. 2 shows how CBCT imaging acquires multiple radiographic projection images for reconstruction of the 3D volume image. X-ray source 110 and detector 122 revolve about patient 12 to acquire a 2D projection image at each of a number of rotational angles about axis Q. Reconstruction algorithms, such as filtered back projection (FBP) or other technique, apply the information from each projection image in order to generate a 3D image volume.

CBCT imaging apparatus and the imaging algorithms used to obtain 3D volume images using such systems are well known in the diagnostic imaging art and are, therefore, not described in detail in the present application. Some exemplary algorithms and approaches for forming 3D volume images from the source 2D images, projection images that are obtained in operation of the CBCT imaging apparatus can be found, for example, in the teachings of U.S. Patent No. 5,999,587 entitled "Method of and System for Cone-Beam Tomography Reconstruction" to Ning et al. and of U.S. Patent No. 5,270,926 entitled "Method and Apparatus for Reconstructing a Three-Dimensional Computerized Tomography (CT) Image of an Object from Incomplete Cone Beam Data" to Tam.

In typical applications, a computer or other type of dedicated logic processor act as control logic processor for obtaining, processing, and storing image data is part of the CBCT system, along with one or more displays for viewing image results, as shown in FIG. 1. A computer-accessible memory 132 is also provided, which may be a memory storage device used for longer term storage, such as a device using magnetic, optical, or other data storage media. In addition, the computer-accessible memory can comprise an electronic memory such as a random access memory (RAM) that is used for shorter term storage, such as employed to store a computer program having instructions for controlling one or more computers to practice the method according to methods of the present disclosure.

Overview of dental surface contour imaging apparatus

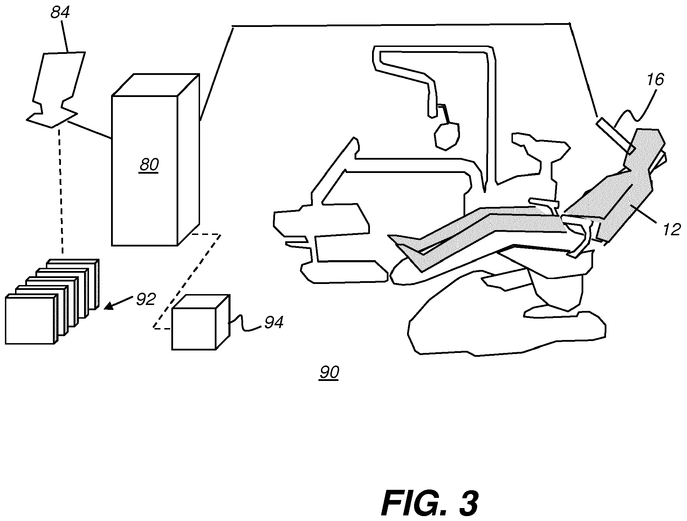

The schematic diagram of FIG. 3 shows a surface contour imaging apparatus 90 for obtaining a 3D view from a succession of reflectance images according to an embodiment of the present disclosure. A camera 16, typically a handheld digital camera, a color depth camera, handheld 3D scanner, or intra-oral 3D scanner, is scanned through the mouth of patient 12 for acquiring a set having multiple reflectance images and associated depth information. A control logic processor 80, in signal communication with camera 16, obtains image data from camera 16 and processes this image data along with depth information in order to generate individual 3D views 92. Control logic processor 80 then combines the scanned 3D views in order to generate and display, on a display 84, a composite 3D surface 94. Error detection and correction logic on control logic processor 80 then operates to identify mis-matched or poorly matched 3D views 92 and generate and display a corrected composite 3D surface 94.

Mesh generation from surface contour imaging

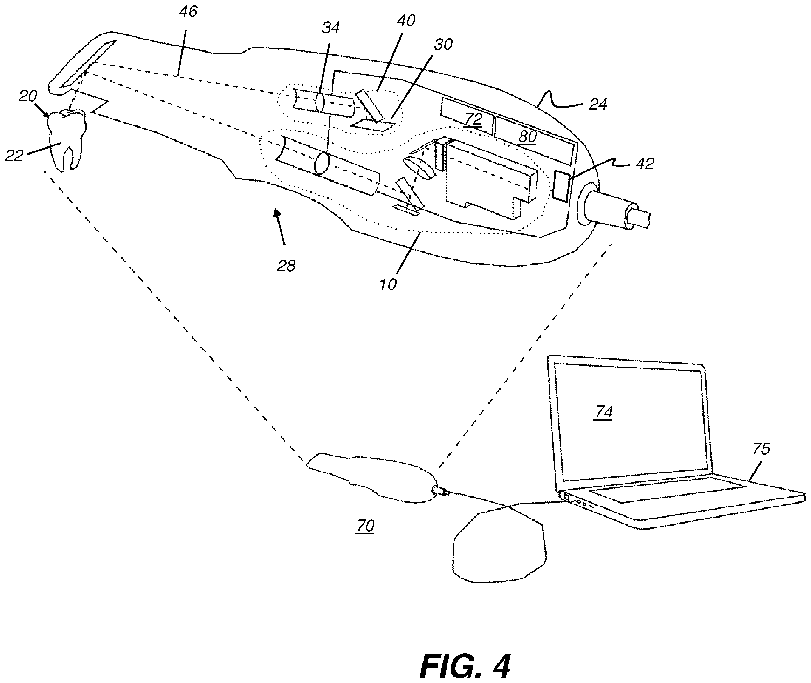

FIG. 4 is a schematic diagram showing an imaging apparatus 70 that can operate as a video camera 24 for polychromatic reflectance image data capture as well as a scanner 28 for executing related projecting and imaging functions used to characterize surface contour with structured light patterns 46. A handheld imaging apparatus 70 uses a video camera 24 for image acquisition for both contour scanning and image capture functions according to an embodiment of the present disclosure.

Control logic processor 80, or other type of computer that may be part of camera 24, controls the operation of an illumination array 10 that generates the structured light and directs the light toward a surface position and controls operation of an imaging sensor array 30. Image data from surface 20, such as from a tooth 22, is obtained from imaging sensor array 30 and stored as video image data in a memory 72. Imaging sensor array 30 is part of a sensing apparatus 40 that includes an objective lens 34 and associated elements for acquiring video image content. Control logic processor 80, in signal communication with camera 24 components that acquire the image, processes the received image data and stores the mapping in memory 72. The resulting image from memory 72 is then optionally rendered and displayed on a display 74, which may be part of another computer 75 used for some portion of the processing described herein. Memory 72 may also include a display buffer. One or more sensors 42, such as a motion sensor, can also be provided as part of scanner 28 circuitry.

In structured light imaging, a pattern of lines or other shapes is projected from illumination array 10 toward the surface of an object from a given angle. The projected pattern from the illuminated surface position is then viewed from another angle as a contour image, taking advantage of triangulation in order to analyze surface information based on the appearance of contour lines. Phase shifting, in which the projected pattern is incrementally shifted spatially for obtaining additional measurements at the new locations, is typically applied as part of structured light imaging, used in order to complete the contour mapping of the surface and to increase overall resolution in the contour image.

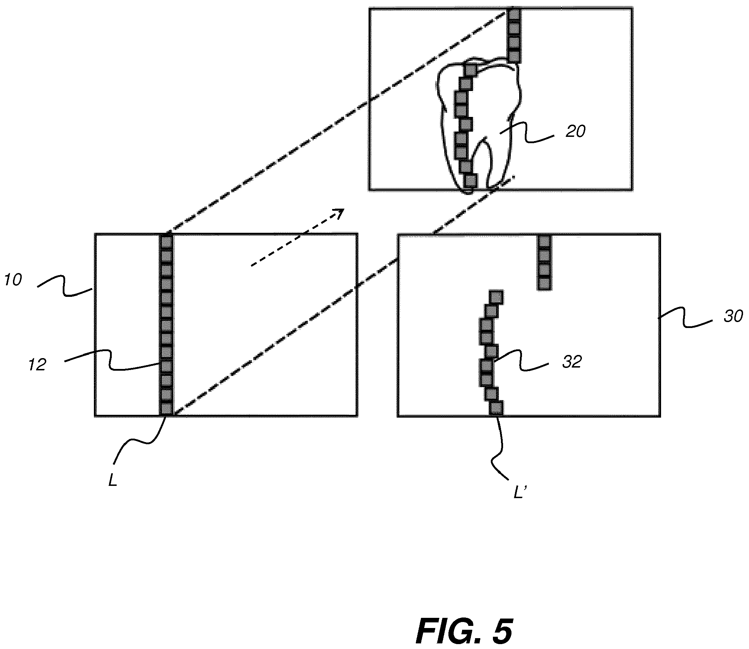

The schematic diagram of FIG. 5 shows, with the example of a single line of light L, how patterned light is used for obtaining surface contour information by a scanner using a handheld camera or other portable imaging device. A mapping is obtained as an illumination array 10 directs a pattern of light onto a surface 20 and a corresponding image of a line L' is formed on an imaging sensor array 30. Each pixel 32 on imaging sensor array 30 maps to a corresponding pixel 12 on illumination array 10 according to modulation by surface 20. Shifts in pixel position, as represented in Figure 5, yield useful information about the contour of surface 20. It can be appreciated that the basic pattern shown in FIG. 5 can be implemented in a number of ways, using a variety of illumination sources and sequences for light pattern generation and using one or more different types of sensor arrays 30. Illumination array 10 can utilize any of a number of types of arrays used for light modulation, such as a liquid crystal array or digital micromirror array, such as that provided using the Digital Light Processor or DLP device from Texas Instruments, Dallas, TX. This type of spatial light modulator is used in the illumination path to change the light pattern as needed for the mapping sequence.

By projecting and capturing images that show structured light patterns that duplicate the arrangement shown in FIG. 5 multiple times, the image of the contour line on the camera simultaneously locates a number of surface points of the imaged object. This speeds the process of gathering many sample points, while the plane of light (and usually also the receiving camera) is laterally moved in order to "paint" some or all of the exterior surface of the object with the plane of light.

A synchronous succession of multiple structured light patterns can be projected and analyzed together for a number of reasons, including to increase the density of lines for additional reconstructed points and to detect and/or correct incompatible line sequences. Use of multiple structured light patterns is described in commonly assigned U.S. Patent Application Publications No. US2013/0120532 and No. US2013/0120533, both entitled "3D INTRAORAL MEASUREMENTS USING OPTICAL MULTILINE METHOD" and incorporated herein in their entirety.

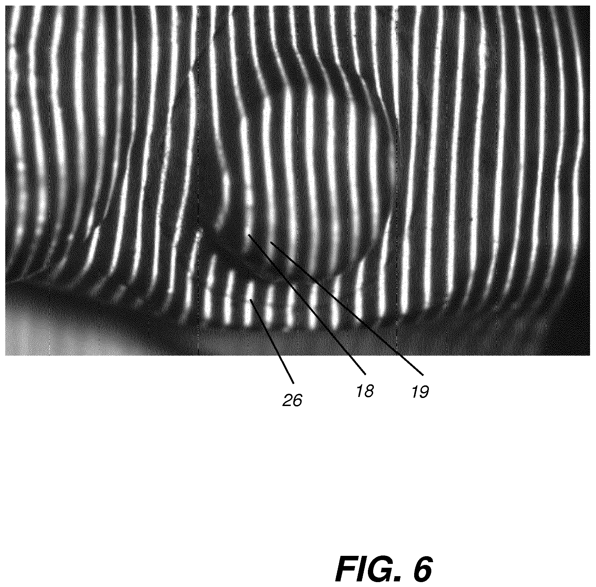

FIG. 6 shows surface imaging using a pattern with multiple lines of light. Incremental shifting of the line pattern and other techniques help to compensate for inaccuracies and confusion that can result from abrupt transitions along the surface, whereby it can be difficult to positively identify the segments that correspond to each projected line. In FIG. 6, for example, it can be difficult over portions of the surface to determine whether line segment 26 is from the same line of illumination as line segment 18 or adjacent line segment 19.

By knowing the instantaneous position of the camera and the instantaneous position of the line of light within an object-relative coordinate system when the image was acquired, a computer and software can use triangulation methods to compute the coordinates of numerous illuminated surface points relative to a plane. As the plane is moved to intersect eventually with some or all of the surface of the object, the coordinates of an increasing number of points are accumulated. As a result of this image acquisition, a point cloud of vertex points or vertices can be identified and used to represent the extent of a surface within a volume. The points in the point cloud then represent actual, measured points on the three-dimensional surface of an object. A mesh can then be constructed, connecting points on the point cloud as vertices that define individual congruent polygonal faces (typically triangular faces) that characterize the surface shape. The full 3D image model can then be formed by combining the surface contour information provided by the mesh with polychromatic image content obtained from a camera, such as camera 24 that is housed with camera 24 in the embodiment described with reference to FIG. 4. Polychromatic image content can be provided in a number of ways, including the use of a single monochrome imaging sensor with a succession of images obtained using illumination of different primary colors, one color at a time, for example. Alternately, a color imaging sensor could be used.

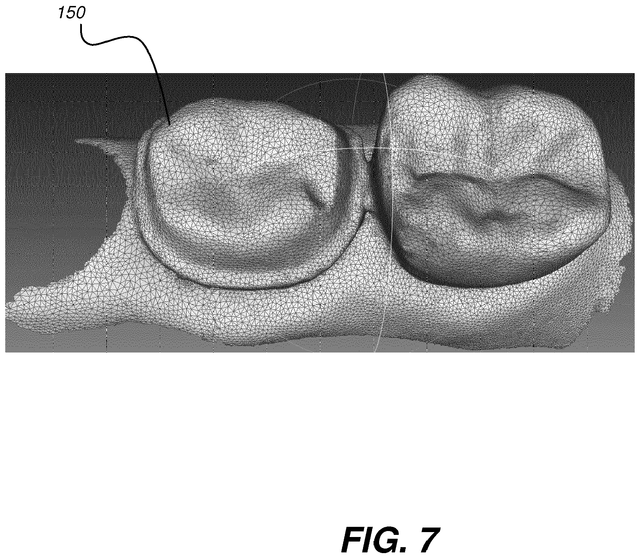

Image processing at control logic processor 80 then generates, from the individual line scan data, a contour surface model. FIG. 7 shows a mesh generated from a succession of structured light images. Further processing of the mesh or point cloud content can be used to generate a mesh as an alternative contour surface model.

It should be noted that other types of reflectance imaging can be used for obtaining intraoral surface contour data. Surface contour information can be obtained using time-of-flight imaging or range imaging methods, such as structure-from-motion processing, for example.

Alternately, 3D surface imaging can be provided for obtaining a mesh or point cloud image using reflectance imaging, with images acquired at video or near-video rates. Each image acquires a portion of the 3D surface; stitching processes can then be used to form a larger surface contour using the acquired image content.

Alternately, it should also be noted that an impression of the patient dentition can also be scanned using a CBCT for obtaining the surface contour image of the patient dentition.

### Image types obtained

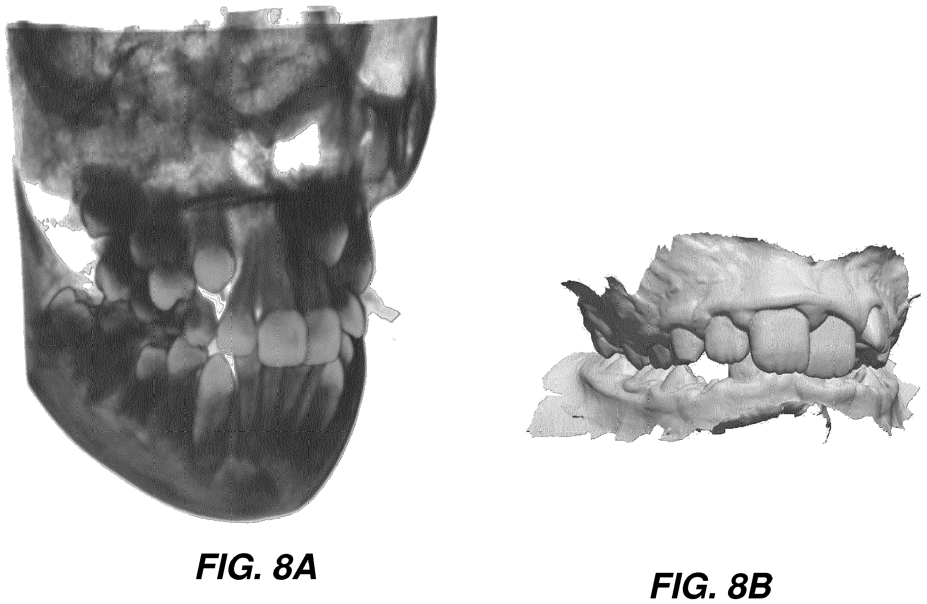

The CBCT imaging apparatus of FIG. 1 acquires 2D projection images and processes these images in order to reconstruct a 3D volume image, as shown in the example of FIG. 8A. The CBCT volume shows the surface as well as inner portions of the dental anatomy and also allows display of a slice through the volume from a viewer-selected angle.

The output of contour imaging apparatus 90 is the mesh or point cloud image, such as that shown in the example of FIG. 8B. The mesh and point cloud image types are similar; the mesh image uses the point cloud and, for surface representation, shows a network of interconnecting lines between points in the point cloud. The mesh pattern is typically triangular, but can alternately use more than three vertices for its basic geometric unit.

In an embodiment of the invention, the 3D mesh of FIG. 8B is a registered 3D mesh. The registered 3D mesh is provided by obtaining a first 3D mesh comprising at least a portion of the maxillary jaw, the first 3D mesh comprising at least a vestibular part and lingual part; obtaining a second 3D mesh comprising at least a portion of the mandibular jaw, the second 3D mesh comprising at least a vestibular part and a lingual part; registering the first 3D mesh with the second 3D mesh in an occlusion position to form the registered 3D mesh. Optionally, at least one of the first and second 3D mesh is segmented.

In another embodiment, the registered 3D mesh is segmented. At least one tooth may be segmented.

An embodiment of the present invention allows the two different types of images shown for a patient in FIGs. 8A and 8B to be accurately registered to each other.

Mesh patterns obtained from intra-oral scanning can be used to show the maxillary and mandibular jaws registered in occlusion, as shown in FIG. 8B.

### Registration of the Surface Contour Mesh to the 3D Volume

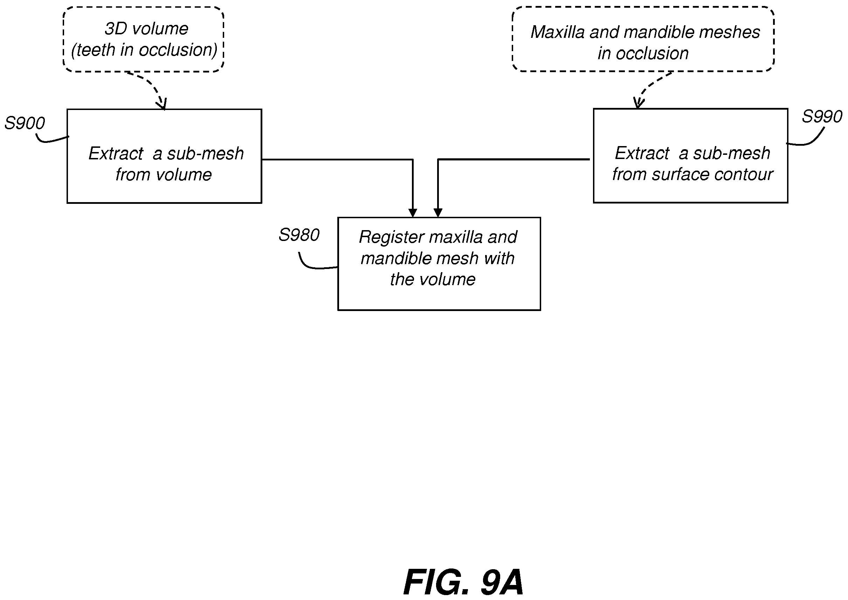

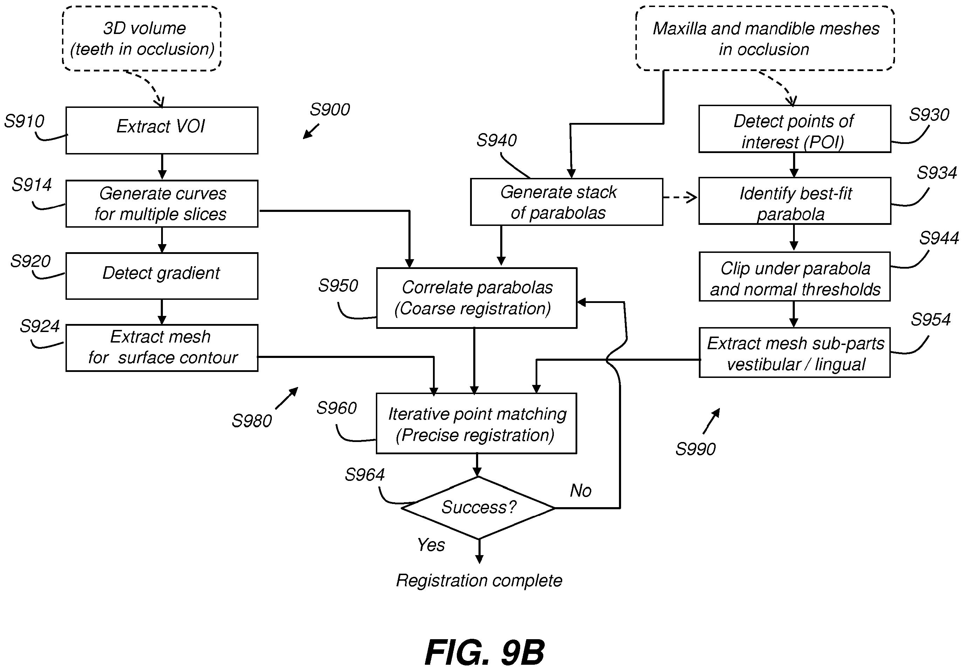

The logic flow diagrams of FIGs. 9A and 9B show a sequence for registering a surface mesh or point cloud for the patient with the corresponding 3D volume image reconstruction from CBCT. FIG. 9A gives an overview of the registration sequence. A sub-mesh extraction sequence S990 extracts a sub-mesh from the surface contour image obtained using surface contour imaging apparatus 90 of FIG. 3, also with the teeth in occlusion. A sub-mesh extraction sequence S900 extracts a corresponding sub-mesh from the 3D volume obtained with teeth in occlusion from CBCT imaging apparatus 100 of FIG. 1. A registration sequence S980 then executes the processing needed to register the two sub-meshes to each other.

The logic flow diagram of FIG. 9B expands upon the steps for each of the corresponding sequences S900, S980 and S990. Processing along the left portion of the FIG. 9B logic flow diagram relates to sequence S900, extracting the sub-mesh from the reconstructed 3D volume obtained from CBCT imaging, as described previously with reference to FIGs. 1 and 2. Processing along the right-most column of FIG. 9B relates to extraction of sub-mesh in sequence S990 from the surface contour mesh or point cloud generated using the structured light imaging apparatus described previously with reference to FIGs. 3 - 7. For CBCT image processing, images are acquired with the patient's teeth in occlusion. For the scanned contour image, scanning of maxilla and mandible portions of the mouth is executed in the normal sequence, with the patient's mouth open. Teeth meshes are then positioned with the teeth in occlusion using the scan technique of registration. Positioning in occlusion reduces sensitivity to height registration error and helps to decrease registration complexity and computation time.

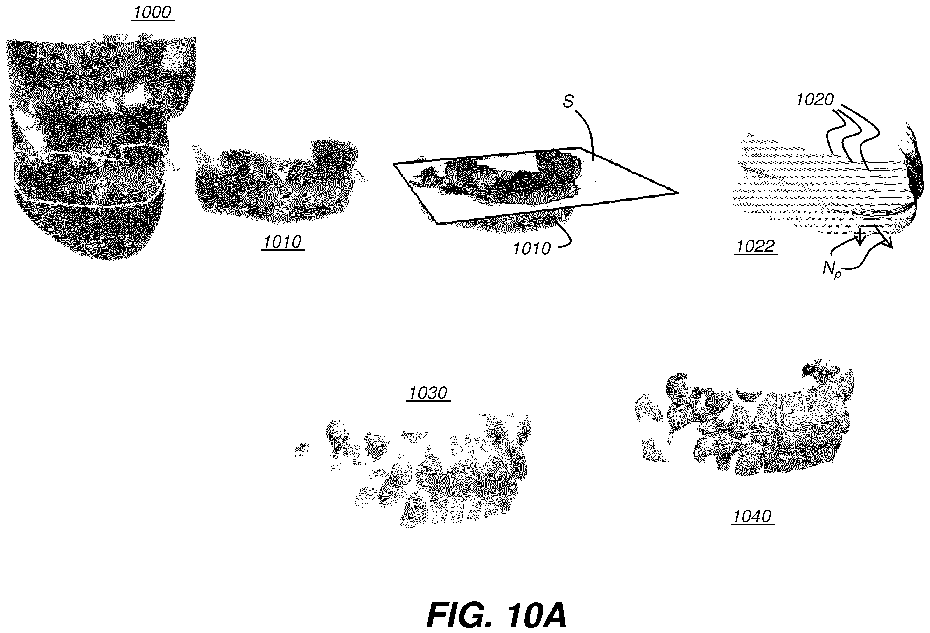

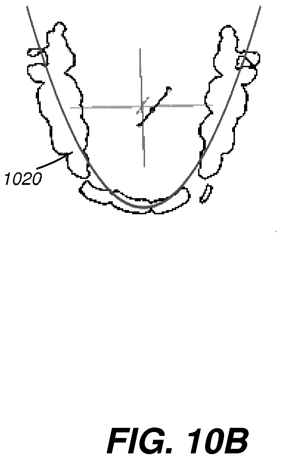

FIG. 10A shows features of the sub-mesh extraction sequence described in the detailed sequence shown in FIG. 9B. In a volume of interest (VOI) extraction step S910, a VOI 1010 is extracted from the full CBCT image volume 1000. The VOI can contain at least a portion of the jaw. A histogram analysis of the volume image can be used in order to differentiate the teeth structures of the maxillary and mandibular from the volume data for defining the volume of interest (VOI). VOI extraction can alternately use conventional segmentation techniques familiar to those skilled in the volume imaging art. A curve generation step S914 generates a parabola 1020 or other continuous curve for each of a set of slices obtained from the VOI. The continuous curve that is generated is twice-differentiable at a plurality of points. The continuous curves that is generated may also be twice-differentiable in each point. Multiple parabolas 1020 are stacked in a spatial ordering, shown as a stack 1022 in FIG. 10A. FIG.

10B shows a single parabola 1020 from a slice, shown in outline form. The single parabola fits the dentition. A gradient detection step S920 then determines for each voxel of the slice the geometric parabola normal $N_{VOI}$ from parabola 1020 and computes, from the collected data, a gradient 1030 along the normal direction in order to detect the corresponding points of surface contour from the VOI. A contour filter is used to obtain the surface contour. A mesh extraction step S924 collects the detected points and forms a mesh corresponding to the gradient to generate a surface contour 1040. Thresholding can be applied in order to identify contour points for the mesh. The vestibular surface contour 1040 is shown. More detailed information on parabola construction is given subsequently.

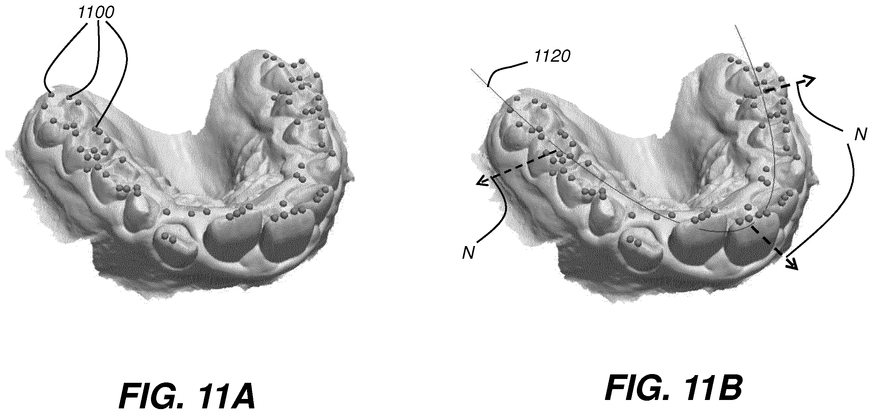

Continuing with the detailed FIG. 9B process, steps along the far right in the logic flow diagram can be executed separately for the mandible and maxilla. A point of interest (POI) detection step S930 identifies distinct features of interest along each jaw surface. The perspective view of FIG. 11A shows multiple points of interest 1100 along a surface contour point cloud or mesh image of the maxilla. In the example shown, local maxima or cusps serve as points of interest 1100. In a parabola fitting step S934, as shown in FIG. 11B, a parabola 1120 is calculated using the identified POIs 1100. Curve calculation for parabola fitting can use least-squares or other curve construction algorithm familiar to those skilled in the image processing art.

An extraction step S944 performs a sub-mesh extraction, in which points of the jaw surface are selected according to two criteria:

(i) Mesh normals must correspond to parabola normals $N_p$. Mesh normals are directed toward the exterior of the mesh and are orthogonal to the mesh curvature. Parabola normals $N_p$ are directed toward the outside of the parabola and are orthogonal to the parabola curvature.
(ii) Mesh points must have a suitable height with respect to the parabola. The relative height of the parabola 1120 corresponds to POI average height.

For normal correspondence criteria, mesh points are selected with an angular tolerance between mesh point normals and corresponding parabola normals $N_p$, such as within 60 degrees, for example. For height selection, a portion of the contour surface extending from the parabola is clipped. For the maxillary structures, this portion lies below the parabola 1120, such as up to 6mm below, for example.

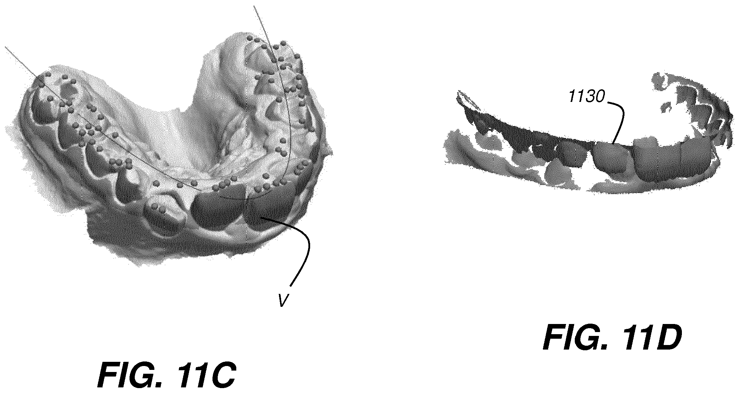

FIG. 11C shows an example with vestibular surface portions V of the maxillary mesh or point cloud identified. In a mesh extraction step S954, the corresponding vestibular and lingual meshes are extracted. FIG. 11D shows vestibular meshes extracted from both mandible and maxilla.

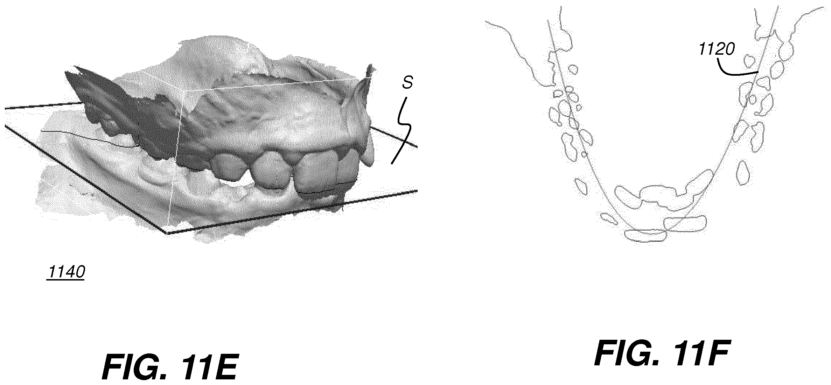

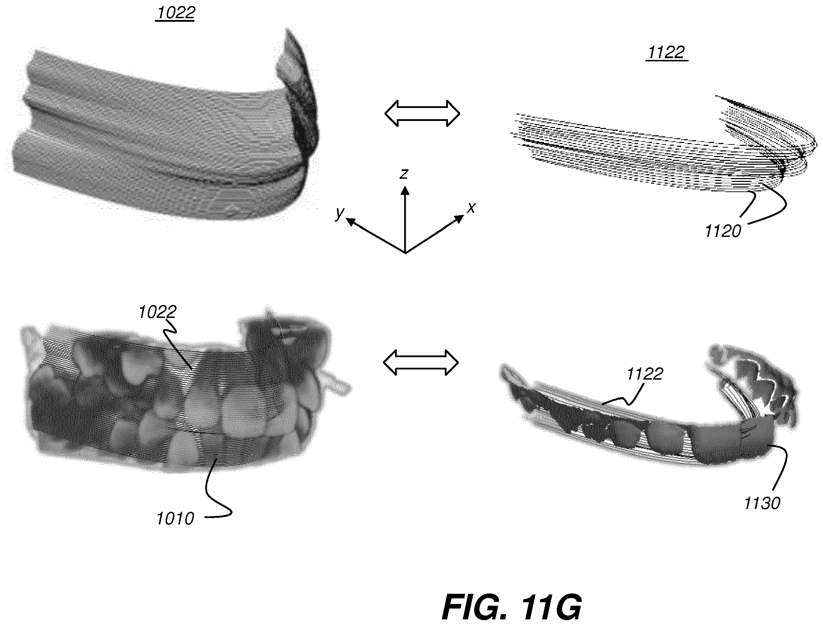

Now referring to steps in registration sequence S980 of FIGs. 9A and 9B, a parabola generation step S940 forms a stack of parabolas for a combined mesh with both jaws. Using the pattern shown for a single slice S in FIG. 11E, a succession of slices S can be stacked on a combined mesh 1140, preferably using the same spacing employed for generating the slice stack for the volume image in step S914. FIG. 11F shows a single parabola 1120 for a slice, with the slice features shown in outline form. FIG. 11G shows a stack of parabolas 1122 formed from parabolas 1120 from successive slices. A parabola correlation step S950 correlates the stacked parabolas 1120 from the surface contour mesh or point cloud data to the stacked parabolas 1022 from volume slices to provide a rough, initial registration of the surface contour content with the 3D volume. Using the x, y, z axes assignments shown in FIG. 11G, the correlation logic aligns the parabola peaks or summits along all three axes and provides rotation of the parabola curvature about the z axis. The correlation of the summit points provides a 3D point-to-point correspondence, that is, a 3D translation.

A point matching step S960 then uses a point matching algorithm to provide precise registration of the surface contour data to the 3D volume data by registering the extracted mesh and sub-meshes from both data sets. A decision step S964 can then determine whether or not registration is successful. If not successful, processing relating to the parabola matching of step S950 can be repeated as necessary.

Point-matching step S960 can use a registration tool, such as Iterative Closest Point (ICP) registration, familiar to those skilled in the imaging art. ICP is used for alignment of three dimensional models given initial assumption of the rigid body transformation required. In summary, ICP uses one point cloud (vertex cloud or mesh) as the fixed reference, or target; a second point cloud, the source, is transformed using operations of translation and rotation in order to match the reference. ICP processing iteratively revises the transformation (combination of translation and rotation) needed to minimize an error metric, usually a distance from the source to the reference point cloud, such as the sum of squared differences between coordinates of matched pairs of points or other features.

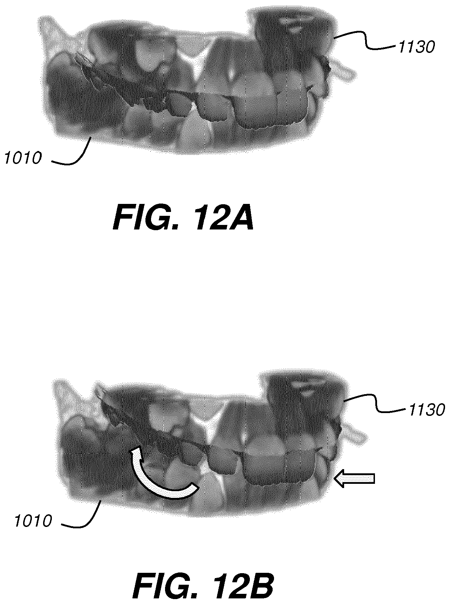

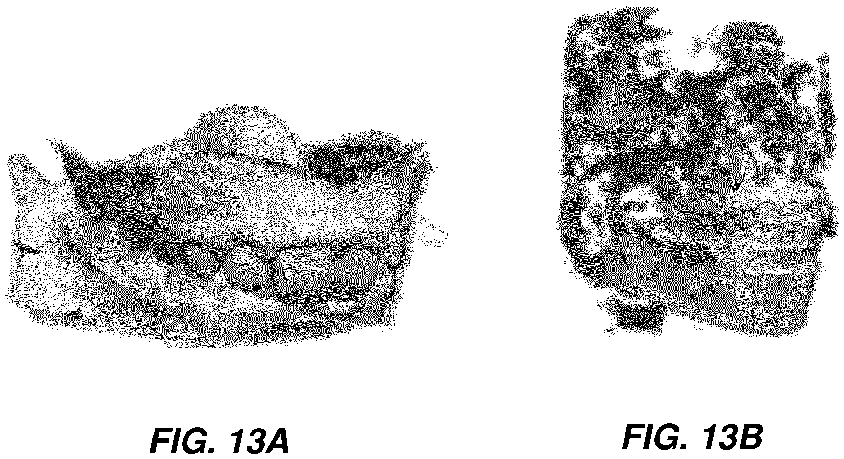

By way of example, FIGs. 12A and 12B show coarse alignment of mesh sub-part 1130 to VOI 1010. Slight translation and rotation, as provided using ICP, are calculated and executed in order to more closely fit the two surface representations to each other. FIGs. 13A and 13B show views of a completed registration of mesh combined vestibular surfaces to volume vestibular surfaces according to an embodiment of the present disclosure.

The method for registering a 3D mesh and a volume image may further comprise at least one of displaying, storing, transmitting the registered 3D mesh with the 3D volume image.

The mandibular jaw and the maxillar jaw of the 3D volume image may also be segmented based on registration of the registered mesh with the generated contour surface content (1040).

Parabola generation

Calculation of parabolas helps to simplify initialization and alignment for embodiments of the present disclosure. Embodiments of the present disclosure also use parabolas for computing normal vectors, for example.

The parabola that fits a particular slice has the equation:

$$x^2 + 2bxy + b^2y^2 + dx + ey + f = 0$$

To compute projection of a point M on the parabola, the simplified equation in the coordinate system rotated from angle $\theta$ and translated toward the peak (or summit) of the parabola can be expressed:

$$Y_t = AX_t^2.$$

For a point $M(x_0, y_0)$, coordinates after rotation and translation are $(x_{t0}, y_{t0})$.

Projection on the parabola is point $P(x_t, y_t)$ where $y_t = Ax_t^2$.

Tangent is the vector $\vec{V_t} \begin{pmatrix} 1 \\ 2Ax_t \end{pmatrix}$ orthogonal to vector $\vec{MP}$.

For orthogonal vectors, $\vec{MP} \cdot \vec{V_t} = 0$

This yields:

$$2Ax_t^3 + (1-2Ayt_0)x_t - xt_0 = 0$$

From which $x_t$ and $y_t = Ax_t^2$ are deduced, giving the position of point P.

Consistent with at least one exemplary embodiment, exemplary methods/apparatus can use a computer program with stored instructions that perform on image data that is accessed from an electronic memory. As can be appreciated by those skilled in the image processing arts, a computer program of an exemplary embodiment herein can be utilized by a suitable, general-purpose computer system, such as a personal computer or workstation. However, many other types of computer systems can be used to execute the computer program of described exemplary embodiments, including an arrangement of one or networked processors, for example.

A computer program for performing methods of certain exemplary embodiments described herein may be stored in a computer readable storage medium. This medium may comprise, for example; magnetic storage media such as a magnetic disk such as a hard drive or removable device or magnetic tape; optical storage media such as an optical disc, optical tape, or machine-readable optical encoding; solid state electronic storage devices such as random-access memory (RAM), or read only memory (ROM); or any other physical device or medium employed to store a computer program. Computer programs for performing exemplary methods of described embodiments may also be stored on computer readable storage medium that is connected to the image processor by way of the internet or other network or communication medium. Those skilled in the art will further readily recognize that the equivalent of such a computer program product may also be constructed in hardware.

It should be noted that the term "memory", equivalent to "computer-accessible memory" in the context of the present disclosure, can refer to any type of temporary or more enduring data storage workspace used for storing and operating upon image data and accessible to a computer system, including a database, for example. The memory could be non-volatile, using, for example, a long-term storage medium such as magnetic or optical storage. Alternately, the memory could be of a more volatile nature, using an electronic circuit, such as random-access memory (RAM) that is used as a temporary buffer or workspace by a microprocessor or other control logic processor device. Display data, for example, is typically stored in a temporary storage buffer that can be directly associated with a display device and is periodically refreshed as needed in order to provide displayed data. This temporary storage buffer can also be considered to be a memory, as the term is used in the present disclosure. Memory is also used as the data workspace for executing and storing intermediate and final results of calculations and other processing. Computer-accessible memory can be volatile, non-volatile, or a hybrid combination of volatile and non-volatile types.

It will be understood that computer program products for exemplary embodiments herein may make use of various image manipulation algorithms and/or processes that are well known. It will be further understood that exemplary computer program product embodiments herein may embody algorithms and/or processes not specifically shown or described herein that are useful for implementation. Such algorithms and processes may include conventional utilities that are within the ordinary skill of the image processing arts. Additional aspects of such algorithms and systems, and hardware and/or software for producing and otherwise processing the images or co-operating with the computer program product of the present disclosure, are not specifically shown or described herein and may be selected from such algorithms, systems, hardware, components and elements known in the art.

Exemplary embodiments according to the present disclosure can include various features described herein (individually or in combination).

While the invention has been illustrated with respect to one or more implementations, alterations and/or modifications can be made to the illustrated examples without departing from the spirit and scope of the appended claims. In addition, while a particular feature of the invention can have been disclosed with respect to only one of several implementations/exemplary embodiments, such feature can be combined with one or more other features of the other implementations/exemplary embodiments as can be desired and advantageous for any given or particular function. The term "a" or "at least one of' is used to mean one or more of the listed items can be selected. The term "about" indicates that the value listed can be somewhat altered, as long as the alteration does not result in failure of the process or structure to conform to the described exemplary embodiment.

The invention has been described in detail with particular reference to a presently preferred embodiment, but it will be understood that variations and modifications can be effected within the spirit and scope of the invention.

The presently disclosed embodiments are therefore considered in all respects to be illustrative and not restrictive. The scope of the invention is indicated by the appended claims, and all changes that come within the meaning and range of equivalents thereof are intended to be embraced therein.

**Claims**

1. A method for registering a 3D mesh and a 3D volume image of a patient's teeth comprising:

   a) obtaining a 3D volume image of the patient's teeth in an occlusion position, the 3D volume image containing at least one jaw portion;
   b) generating contour surface content corresponding to at least one of lingual and vestibular surfaces of the teeth from the 3D volume image;
   c) providing a registered 3D mesh in an occlusion position, the registered mesh comprising at least a portion of a maxillary jaw and a mandibulary jaw, each of the jaws comprising at least one of the vestibular and lingual surfaces;
   d) registering the registered 3D mesh with the generated contour surface content.

2. A method for registering a 3D mesh and a 3D volume image of a patient's teeth further comprising at least one of displaying, storing, transmitting the registered 3D mesh with the 3D volume image.

3. The method of claim 1 wherein the 3D volume image is obtained from a computed tomography apparatus.

4. The method of claim 1 wherein providing the registered 3D mesh comprises:

   - obtaining a first 3D mesh comprising at least a portion of the maxillary jaw, the first 3D mesh comprising at least a vestibular part and a lingual part;
   - obtaining a second 3D mesh comprising at least a portion of the mandibular jaw, the second 3D mesh comprising at least a vestibular part and a lingual part;
   - registering the first 3D mesh with the second 3D mesh in an occlusion position to form the registered 3D mesh.

5. The method of claim 4 wherein at least one of the first and second 3D meshes are obtained using an intraoral scanner.

6. The method of claim 1 further comprising the step of identifying a volume of interest including at least one jaw portion from content of the 3D volume image.

7. The method according to claim 1 wherein the registered 3D mesh is segmented.

8. The method of any of preceding claims further comprising segmenting the mandibular jaw or the maxillary jaw of the 3D volume image based on registration step d).

9. The method according to any of the preceding claims, further comprising the step of defining a first set of continuous curves corresponding to tooth position on the 3D volume image.

10. The method according to claim 8 wherein the generated contour surface content corresponding to at least one of either lingual or vestibular surfaces of the teeth is generated based on the first set of continuous curves.

11. The method according to claim 1 further comprising defining a second set of continuous curves for the registered 3D mesh.

12. The method according to claim 10 further comprising extracting a lingual or vestibular portion of the registered 3D mesh based on the second set of continuous curves.

13. The method according to claim 9 to 12, wherein the continuous curves are parabolas.

14. The method according to claim 9 wherein defining one of the first and second set of continuous curves comprises defining a set of parallel curves.

15. The method according to claim 1 wherein registering the registered 3D mesh with the generated contour surface content comprises a first registration based on the first and second set of curves and a second registration based on the vestibular or lingual surfaces.

FIG. 1

**FIG. 2**

**FIG. 3**

**FIG. 4**

EP 3 675 036 A1

**FIG. 5**

EP 3 675 036 A1

FIG. 6

FIG. 7

*FIG. 8B*

*FIG. 8A*

```
  ┌ ─ ─ ─ ─ ─ ─ ─ ─ ─ ─ ┐                          ┌ ─ ─ ─ ─ ─ ─ ─ ─ ─ ─ ─ ─ ─ ─ ─ ┐
  │   3D volume          │                          │  Maxilla and mandible meshes   │
  │ (teeth in occlusion) │                          │         in occlusion           │
  └ ─ ─ ─ ─ ─ ─ ─ ─ ─ ─ ┘                          └ ─ ─ ─ ─ ─ ─ ─ ─ ─ ─ ─ ─ ─ ─ ─ ┘
```

S900  → Extract a sub-mesh from volume

S990 → Extract a sub-mesh from surface contour

S980 → Register maxilla and mandible mesh with the volume

**FIG. 9A**

**FIG. 9B**

**FIG. 10A**

EP 3 675 036 A1

FIG. 10B

**FIG. 11A**

**FIG. 11B**

EP 3 675 036 A1

FIG. 11C

FIG. 11D

S

1140

**FIG. 11E**

1120

**FIG. 11F**

FIG. 11G

FIG. 12A

FIG. 12B

*FIG. 13B*

*FIG. 13A*

Europäisches
Patentamt

European
Patent Office

Office européen
des brevets

**EUROPEAN SEARCH REPORT**

Application Number

EP 18 30 6869

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y<br>A | WO 2017/218040 A1 (CARESTREAM HEALTH INC [US]) 21 December 2017 (2017-12-21)<br>* abstract; figures 10C, 36 *<br>* page 4, line 1 - line 13 *<br>* page 20, line 17 - line 03 *<br>* page 33, line 30 - page 34, line 8 *<br>* page 35, line 7 - line 9 *<br>----- | 1,3,6,7,9<br>2,8<br>4,5,10-15 | INV.<br>G06T7/30 |
| X<br><br><br><br><br><br><br><br><br><br><br><br>Y<br>A | NILSSON JOHANNA ET AL: "Virtual bite registration using intraoral digital scanning, CT and CBCT:In vitroevaluation of a new method and its implication for orthognathic surgery",<br>JOURNAL OF CRANIO-MAXILLO-FACIAL SURGERY, CHURCHILL LIVINGSTONE, GB,<br>vol. 44, no. 9, 23 June 2016 (2016-06-23), pages 1194-1200, XP029724484,<br>ISSN: 1010-5182, DOI:<br>10.1016/J.JCMS.2016.06.013<br>* abstract; figure 5 *<br>* sections 1. Introduction; 2. Materials and methods *<br>----- | 1-3,6<br><br><br><br><br><br><br><br><br><br><br><br>2<br>4,5,10-15 | |
| X<br><br><br><br><br><br><br><br><br><br><br>Y<br>A | HERNÁNDEZ-ALFARO F ET AL: "New protocol for three-dimensional surgical planning and CAD/CAM splint generation in orthognathic surgery: an in vitro and in vivo study",<br>INTERNATIONAL JOURNAL OF ORAL AND MAXILLOFACIAL SURGERY, COPENHAGEN, DK,<br>vol. 42, no. 12, 13 June 2013 (2013-06-13), pages 1547-1556, XP028780892,<br>ISSN: 0901-5027, DOI:<br>10.1016/J.IJOM.2013.03.025<br>* abstract; figure 12 *<br>* section: Part I: in vitro study; first two columns of page 1554 *<br>----- | 1,3,6,8<br><br><br><br><br><br><br><br><br><br><br>8<br>4,5,10-15 | TECHNICAL FIELDS SEARCHED (IPC)<br><br>G06T |

-/--

The present search report has been drawn up for all claims

| Place of search | Date of completion of the search | Examiner |
|---|---|---|
| Munich | 3 July 2019 | Rimassa, Simone |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

page 1 of 2

Europäisches
Patentamt

European
Patent Office

Office européen
des brevets

## EUROPEAN SEARCH REPORT

Application Number

EP 18 30 6869

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 105 447 908 A (SHANDONG HOTEAM SOFTWARE CO LTD) 30 March 2016 (2016-03-30) * abstract * ----- | 1-15 | |

TECHNICAL FIELDS SEARCHED (IPC)

The present search report has been drawn up for all claims

| Place of search | Date of completion of the search | Examiner |
|---|---|---|
| Munich | 3 July 2019 | Rimassa, Simone |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

EP 3 675 036 A1

## ANNEX TO THE EUROPEAN SEARCH REPORT
## ON EUROPEAN PATENT APPLICATION NO.

EP 18 30 6869

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2019

| Patent document cited in search report | Publication date | Patent family member(s) | Publication date |
|---|---|---|---|
| WO 2017218040 A1 | 21-12-2017 | EP    3471617 A1<br>WO 2017218040 A1 | 24-04-2019<br>21-12-2017 |
| CN 105447908 A | 30-03-2016 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8670521 B
- US 5999587 A
- US 5270926 A
- US 20130120532 A
- US 20130120533 A